# EUROPEAN PATENT APPLICATION

(11) **EP 4 386 382 A1**
(43) Date of publication of application: **19.06.2024**
(21) Application number: 22214000.6
(22) Date of filing: 15.12.2022
(51) Int. Cl.: G01N 33/493

(54) **DEVICE, SYSTEM AND METHOD FOR AUTOMATED URINE ANALYSIS**

(71) Applicant: Stichting IMEC Nederland, 6708 WH Wageningen (NL)
(72) Inventor: Wentink, Eva, 3001 Leuven (BE); Zhang, Xu, 3001 Leuven (BE); Zentile, Mark, 3001 Leuven (BE); Kho, Esther, 3001 Leuven (BE)
(74) Representative: Patent Department IMEC

(57) **Abstract**

A device (102) for urinalysis in a bowl of a toilet (104) is presented. The device (102) includes a cassette (116) comprising a dipstick (202) on a roll. Also, the dipstick (202) has a sequence of sensing area (206) and non-sensing area (208) on the roll. Further, the device (102) includes a control unit (302) operatively coupled to the cassette (116). The control unit (302) is configured to automatically feed the sensing area (206) of the dipstick (202) to a predetermined location so that urine flows over the sensing area (206) of the dipstick (202) to induce a reaction on the sensing area (206). In addition, the device (102) includes a sensor unit (304) configured to measure the at least one value of the sensing area (206) that reacted with the urine and, transmit the measured at least one value to an external device (106).

## Description

### BACKGROUND

Embodiments of the present specification relate generally to urinalysis, and more particularly to a device, system and method for an automated urine analysis in a toilet.

Urine analysis has become more and more popular as it is a non-invasive test that can give feedback on the health status of a subject. In general, urine analysis is performed only when a subject has health issues or complaints, and he/she is advised by a doctor or medical practitioner. However, it is beneficial to perform urine analysis on a regular basis so as to detect early health issues. Typically, urine analysis may be performed at home by using a dipstick. This is an easy method where the subject urinates over the dipstick and waits for two minutes to see if there is any reaction induced on the assay of dipstick. Further, color of the assay of interest on the dipstick is compared with reference colors to determine markers of different diseases.

However, this method is not accurate because it is user dependent and not automated. More specifically, the user may have to visually readout the dipstick, which may be prone to error. Otherwise, the user may have to manually stick the wet dipstick in a machine, which is again user dependent and may be prone to user error. Moreover, this method requires the user to hold the dipstick and urinate on the dipstick, which in-turn is uncomfortable and restricts people from using it frequently. Thus, there is a need for system and method to perform urine analysis for a large population and provide their health status in an easy manner.

### SUMMARY

In accordance with aspects of the present specification, a device for urinalysis in a bowl of a toilet is presented. The device includes a cassette comprising a dipstick on a roll. Also, the dipstick has a sequence of sensing area and non-sensing area on the roll. Further, the device includes a control unit operatively coupled to the cassette. The control unit is configured to automatically feed the sensing area of the dipstick to a predetermined location so that urine flows over the sensing area of the dipstick to induce a reaction on the sensing area. In addition, the device includes a sensor unit configured to measure the at least one value of the sensing area that reacted with the urine and, transmit the measured at least one value to an external device. Further, the external device determines one or more parameters associated with the subject.

In accordance with another aspect of the present specification, a system for urinalysis is presented. The system comprises the device and an external device. The external device includes a data transceiver configured to receive at least one value of the sensing area from the sensor unit in the device. Further, the external device includes a processor coupled to the data transceiver and configured to compare the at least one value of the sensing area with a predetermined dipstick values to determine one or more parameters associated with the subject. Also, the external device includes a display unit configured to display the determined one or more parameters associated with the subject.

In accordance with another aspect of the present specification, a method for urinalysis in a bowl of a toilet using a device is presented. The method includes positioning a cassette in a bowl of a toilet, where the cassette comprises a dipstick on a roll. Also, the dipstick has a sequence of sensing area and non-sensing area on the roll. In addition, the method includes automatically feeding the sensing area of the dipstick to a predetermined location so that urine flows over the sensing area of the dipstick to induce a reaction on the sensing area. Further, the method includes measuring at least one value of the sensing area that reacted with the urine. Furthermore, the method includes receiving at least one measured value of the sensing area that is in contact with a urine sample of a subject. In addition, the method includes determining one or more parameters associated with the subject based on the received at least one measured value of the sensing area.

### SHORT DESCRIPTION OF THE DRAWINGS

These and other features, aspects, and advantages of the present disclosure will become better understood when the following detailed description is read with reference to the accompanying drawings in which like characters represent like parts throughout the drawings, wherein:
FIG: 1 is a diagrammatical representation of a system having a device for urinalysis, in accordance with aspects of the present specification;
FIG. 2 is a diagrammatical representation of a cassette with a dipstick rolled out of the cassette, in accordance with aspects of the present specification;
FIG. 3 is a side view of the device shown in Fig. 1, in accordance with aspects of the present specification;
FIG. 4 is a top view of the device shown in Fig. 1, in accordance with aspects of the present specification; and
FIG. 5 is a flow chart illustrating a method for urinalysis and determining one or more parameters associated with the subject, in accordance with aspects of the present specification.

### DETAILED DESCRIPTION

As will be described in detail hereinafter, various embodiments of system and method for collecting and analyzing urine sample of a subject or patient is presented. The system and method aid in automatically reading the dipstick and determining parameters such as, pH, blood, nitrate, protein of the subject/patient. These parameters may be markers of different diseases. With this system and method, there is no need to hold the dipstick while the user urinates or pees over it. Also, there is no need to manually compare the color of the wet dipstick with reference colors for readout of a urine sample. Thus, avoiding user error caused by visual readouts.

In the following specification and the claims, reference will be made to a number of terms, which shall be defined to have the following meanings. The singular forms "a", "an", and "the" include plural references unless the context clearly dictates otherwise.

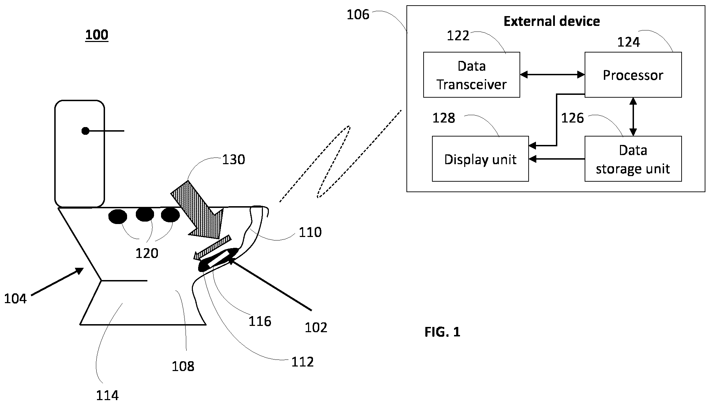

Turning now to the drawings and referring to FIG. 1, a diagrammatical representation of a system 100 for urinalysis, in accordance with aspects of the present specification, is depicted. Urinalysis is defined as a test that aids in assessing one or more aspects of a subject/user's health with a urine sample. Urinalysis is used to detect and manage a wide range of disorders, such as urinary tract infections, kidney disease and diabetes. Typically, urinalysis involves checking the appearance, concentration and content of urine. The system 100 includes a device 102 positioned inside a toilet 104 and an external device 106. The toilet 104 may be a standard or well-known toilet having a bowl 108 to collect urine 130 when a user urinates in the toilet 104. In one example, the toilet may also be a urinal that is specially used for men or women to urinate. The toilet may be of any shape and/or structure that aid the user/subject to urinate. It may be noted that the terms "user" and "subject" may be used interchangeably in the below description. Further, the device 102 may be communicatively coupled to the external device 106 through a short-range or long-range communication network.

In a presently contemplated configuration, the device 102 includes a holder 110 for detachably coupling the device 102 to the bowl 108 of the toilet 104. The holder 110 may be an elongated strip that can be bent at one end to latch it to a rim of the toilet bowl 108 as depicted in FIG. 1. Further, the other end of the elongated strip is connected to a housing 112 of the device 102. The holder 110 may be bent and/or moved to position the device 102 at a desired or predefined location in the toilet bowl 108. More specifically, the device 102 is positioned in such a way that when the user/subject urinates in the toilet 104, the urine 130 first flows over the device 102 and then it is collected at the foot 114 of the bowl 108. In one example, urine or urine sample may flow at least over a top surface of the device 102. It may be noted that the device 102 may be coupled to the toilet 104 by any attaching means such as, vacuum coupler, glue strip, and not limited to the holder 110.

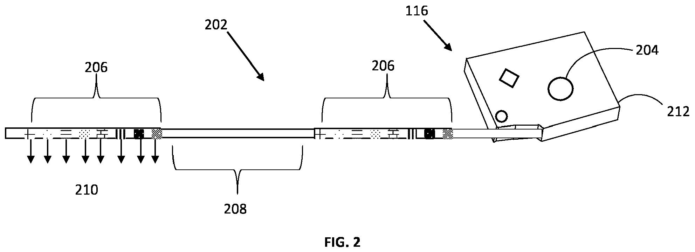

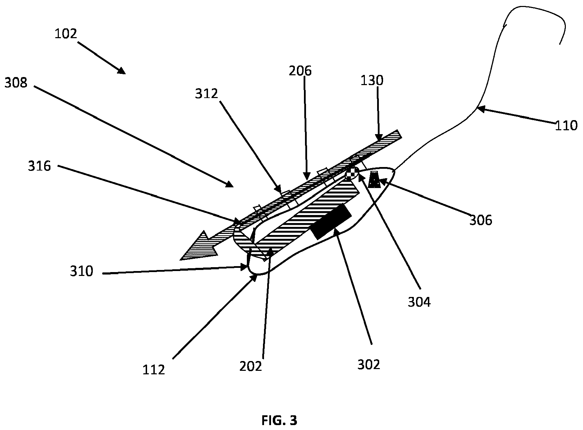

In addition to the holder 110, the device includes a cassette 116, a control unit 302 (see FIG. 3), and a sensor unit 304 (see FIG. 3) that are enclosed in the housing 112. It may be noted that the device 102 may include other components and are not limited to the components shown in FIG. 1. Further, the cassette 116 comprises a dipstick 202 on a roll 204 as depicted in FIG. 2. The cassette 116 may be analogous to a standard or well-known magnetic tape cassette but, the dipstick 202 is rolled and placed on a roll/roller 204 instead of a magnetic tape. Also, the dipstick 202 may be referred to as a tape having a sequence of sensing area 206 and non-sensing area 208 that are rolled over the spool or roller 204 of the cassette 116. As depicted in FIG. 2, a strip of non-sensing area 208 is placed between two consecutive strips of sensing area 206. Further, the sensing area 206 may be referred to as a set of assays 210 on the dipstick that are used for sensing parameters or markers associated with the user/subject. The parameters may include, but not limited to, nitrate, protein, pH values of the user/subject. In one example, the sensing area 206 may include, but not limited to, 11 or 12 assays depending upon the number of parameters/markers to be determined. It may be noted that the terms "assays" and "sensing surfaces" may be used interchangeably in the below description. Moreover, the dipstick 202 is made of biodegradable material which helps in easy flushing of the dipstick 202 after use or readout. It may be noted that the dipstick 202 may also be made of other material and not limited to the biodegradable material.

The sensing area 206 is defined as a part of the dipstick 202 that is sensitive to the content of the urine 130. More specifically, urine flow over the sensing area may induce a reaction on the assays or sensing surfaces 210 of the sensing area 206. As a result, one or more values associated with the sensing area changes. More specifically, one or more sensing surfaces 210 in the sensing area 206 react to the content of the urine and changes its values based on the content. In one example, color values of the sensing surfaces 210 may change based on the content of the urine. It may be noted that the one or more values associated with the sensing area may not change if there is no health issue with the user/subject. Particularly, the sensing surfaces 210 in the sensing area 206 may not react to the content of the urine if the parameters such as pH, protein, nitrate, blood are at their desired/predetermined value or within their desired range of values. Only if there is a substantive deviation in the values of these parameters, the content of the urine would have changed and as a result, the one or more values associated with the sensing area also changes.

In a similar manner, the non-sensing area 208 is defined as a part/strip of the dipstick 202 that is insensitive to the content of the urine. This non-sensing area 208 may act as an intermediate strip between two consecutive sensing areas 206 of the dipstick 202 as depicted in FIG. 2. Also, this strip of non-sensing area 208 is primarily used when the device 102 waits for the next usage. Typically, the dipstick 202 needs to be kept dry as long as possible, hence the sensing area 206 will only be fed out of the cassette 116 once the user/subject is ready for the use of toilet. Until then, only the non-sensing area 208 of the dipstick 202 is kept outside the cassette 116.

Further, the control unit 302 (see FIG. 3) in the device 102 feeds the sensing area 206 of the dipstick 202 to a predetermined location in the toilet 104. The predetermined location may be referred to a location where the subject/user urinates in the toilet 104. In one embodiment, the control unit 302 feeds the sensing area 206 of the dipstick 202 to the predetermined location only when the user is detected and/or the user wants to perform urinalysis. In particular, the control unit 302 may receive a detection signal and/or a subject signal, and in response feeds the sensing area 206 of the dipstick 202 to the predetermined location so that when the subject urinates, the urine flows over the sensing area 206 of the dipstick 202.

In one embodiment, the presence of the subject/user on the toilet 104 or in the vicinity of toilet 104 may be detected by using one or mor auto-detect sensors. The auto-detect sensors 120 may be positioned on or below a toilet seat where the user sits while using the toilet 104. In one example, the auto-detect sensors 120 may be positioned within the device 102. In another example, the auto-detect sensors 120 may be positioned proximate to the toilet 104 for example, at the entrance of the toilet room. The auto-detect sensors 120 may send the detection signal to the control unit 302 when the presence of the user on the toilet 104 or in the vicinity of toilet 104 is detected. In one example, the auto-detect sensors 120 may be weight sensors that senses the weight of the user and accordingly sends the detection signal to the control unit 302. In another example, the auto-detect sensors 120 may be RF-tag sensors that sends RF signal as the detection signal to the control unit 302 when the user is proximate to the toilet 104. The RF-tag sensors may be attached to the user or to a device accompanied by the user.

In another embodiment, the presence of the user on the toilet 104 or in the vicinity of toilet 104 may be detected by using the external device 106 or any other device used by the user. More specifically, the user may install an app (application) in the external device 106. Further, the user may send a subject signal using the app to the control unit 302. The subject signal indicates the presence of the user on/near the toilet 104.

In yet another embodiment, the control unit 302 may feed the sensing area 206 of the dipstick 202 to the predetermined location based on one or more preferences of the user. In particular, the user may specify if the user wants to measure every toilet visit or only on-demand. In one example, the user may use the app in the external device 106 to set his preferences such as to use the device, either on a timer or just before/while going to the toilet. Based on the preferences, the app in the external device 106 sends the subject signal to the control unit 302 to feed the sensing area 206 of the dipstick 202 to the predetermined location.

In one more embodiment, the control unit 302 may feed the sensing area 206 of the dipstick 202 to the predetermined location based on urine flow detection. More specifically, the sensors such as conductivity sensor, temperature sensor, humidity sensor, fluid level sensor are used to detect if the user is actually urinating over the device 102 or if flushing of the toilet has occurred. If the urine flow is detected, these sensors may send a signal to the control unit 302 to feed the sensing area 206 of the dipstick 202 to the predetermined location.

It may be noted that the above-described embodiments of user detection, user preferences, and urine flow detection may be used alone or in combination. More specifically, the control unit 302 may feed the sensing area 206 of the dipstick 202 only when the urine flow is detected, or when the user presence and the urine flow is detected. Similarly, the control unit 302 may also feed the sensing area 206 based on only the user preferences in the app, or in combination of user preferences with the user presence and/or urine flow detection.

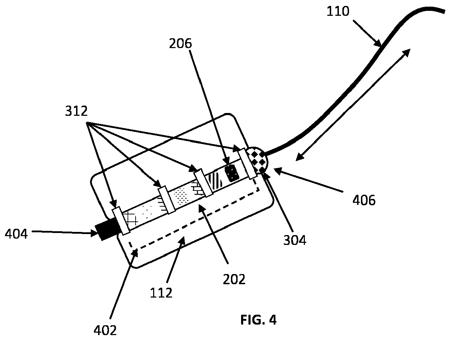

Further, the urine flow may induce reaction over one or more sensing surfaces 210 of the sensing area 206 and this reaction may change values of the sensing surfaces 210. In one example, the values of the sensing surfaces 210 may be referred to as color values of the dipstick 202. Thereafter, the sensor unit 304 reads or measures the one or more values of the sensing area 206 and transmits the measured values to the external device 106 to determine one or more parameters associated with the subject. In one example, the sensor unit 304 may include a color-sensor such as, but not limited to, a camera or an RGB sensor that is used for color sensing of one or more surfaces 210 of the sensing area 206. Further, the parameters may include, but not limited to, pH, protein, nitrate, blood. These parameters may deviate depending upon the content of the urine. This deviation in the parameters may be used to determine diseases. It may be noted that the aspect of feeding the dipstick 202 and sensing the values of the dipstick is explained in greater detail with reference to FIGs. 3 and 4.

In the presently contemplated configuration, the external device 106 includes a data transceiver 122, a processor 124, a data storage unit 126, and a display unit 128. The data transceiver 122 is communicatively coupled to the device 102 in the toilet 104 to receive the measured one or more values of the sensing area 206. Further, these measured values are transmitted to the processor 124.

Thereafter, the processor 124 compares the measured values with a set of reference values stored in the data storage unit 126 to determine one or more parameters of the user/subject. In one example, a look-up table of different reference values and its corresponding parameter are pre-stored in the data storage unit 126. Further, the processor 124 may verify the look-up table to identify the parameter associated with the reference value that matches or proximate to the measured/received value. Upon determining one or more parameters of the user/subject, the processor 124 may determine variation or deviation of the parameter from its initial value or predefined value. This variation or deviation in parameter indicates one or more markers or related diseases of the user/subject. Thereafter, the processor 124 communicates to the user by displaying these parameters, markers, and/or related disease information on the display unit 128. In one embodiment, the processor 124 may provide the feedback or information related to the parameters via the app in the external device 106. It may be noted that processor 124 may also communicate by other means such as audio clips and is not limited to displaying.

Thus, by employing the exemplary system 100, the urinalysis is performed automatically. Also, with this system 100, there is no need to hold the dipstick while the user urinates over it. Also, there is no need to manually compare the color of the wet dipstick with reference colors for readout of a urine sample. Thus, avoiding user error caused by visual readouts.

Referring to FIG. 2, a diagrammatical representation of a cassette with a dipstick rolled out of the cassette, in accordance with aspects of the present specification, is depicted. The cassette 116 contains spool or roll of dipstick 202 housed in a plastic or metal enclosure 212. The dipstick roll 202 may be of biodegradable material. It may be noted that the dipstick 202 may also be made of other material and not limited to the biodegradable material. When the spool or roll 204 is rotated in a clockwise direction, the dipstick 202 begins to roll-out and move along a guided path of the enclosure 212 as depicted in FIG. 2. As the dipstick rolls out, the sequence of sensing area 206 and non-sensing area 208 of the dipstick 202 is guided out of the cassette 116 and fed towards a predetermined location in the toilet 104. In one example, the sensing area 206 of the dipstick 202 is moved to a top surface of the device housing 112 where the urine flows over the device 102.

The sensing area 206 includes a plurality of sensing surfaces 210 that are sensitive to the content of the urine. Also, each of sensing surfaces 210 has a color that is associated with a color value. Further, when a sensing surface reacts with the urine, the color of the respective sensing surface changes. As a result, the color value associated with this sensing surface also changes. This change in the color value is sensed or measured by the sensor unit 304. In one example, color sensor and/or RGB sensor are included in the sensor unit 304 to measure these color values.

Upon measuring the color values, the sensing area 206 of the dipstick 202 is removed from the dipstick 202 and flushed in the toilet 104. In one example, a retractable knife 306 (see FIG. 3) is used to cut the part/strip of the dipstick 202 having the first sensing area 206. Thereafter, only the non-sensing area 208 is placed outside the cassette 116 till the next usage of the toilet 104 is detected. The aspect of removing the sensing area part from the dipstick 202 is explained in greater detail with reference to FIG. 3.

FIG. 3 is a side view of the device 102 shown in Fig. 1, in accordance with aspects of the present specification. The device 102 includes the cassette 116, the control unit 302, the sensor unit 304 that are enclosed in the housing 112. The control unit 302 is operatively coupled to the cassette 116. Also, the control unit 302 is configured to automatically feed the sensing area 206 of the dipstick 202 to a predetermined location. The predetermined location may be at the top surface 308 of the housing 112 as depicted in FIG. 3. In one example, the control unit 302 may include one or more actuators or motors to rotate the spool or roll 204 of the cassette 116 so that the dipstick 202 rolls out of the cassette 116. Further, the sensor unit 304 is positioned at one end of the top surface 308 of the housing 112 to read the values of the sensing area 206.

Also, the housing 112 includes a closing valve 310, one or more shakers 312, and a retractable knife 306 that are positioned as shown in FIG. 3. It may be noted that the closing valve 310, the shakers 312, and the retractable knife 306 may be positioned at any desired location in the device 102 or inside/outside the housing 112. The closing valve 310 is positioned at one end of the housing 112 to allow the dipstick 202 to roll out of the cassette 116 to the predetermined location. The closing valve 310 acts as an opening of the housing 112 where the dipstick 202 exits the housing 112 when it is rolled out. Also, the closing valve 310 is configured to cease urine or water to flow into the device 102. As a result, the dipstick roll 202 inside the device 102 is waterproofed and maintained dry.

Further, the one or more shakers 312 are placed on the top surface 308 of the housing 112 as depicted in FIG. 3. The shakers 312 are configured to remove excess urine or deposited urine on the sensing area 206 of the dipstick 202. More specifically, when the user/subject urinates, the urine 130 flows over the device 102 particularly, on the top surface 308 of the device 102. This urine flow induces reaction on the sensing area 206. Particularly, a portion of the urine 130 reacts with the bottom side 316 of the sensing area 206. Further, the excess urine that are deposited on the top surface 308 or on the sensing area 206 at the top surface 308 is removed by using the shakers 312. In one embodiment, the control unit 302 may send a signal to the shakers 312 to vibrate and remove the excess urine on the device 102. Also, the shakers 312 may be used to position and/or hold the dipstick 202 on the top surface 308 of the housing 112.

Furthermore, the retractable knife 306 is configured to cut off the sensing area 206 of the dipstick after the values of the sensing area 206 are measured by the sensor unit 304. The retractable knife 306 is positioned at an end of the housing 112 where the sensor unit 304 is placed, particularly adjacent or next to the sensor unit 304 as depicted in FIG. 3. The retractable knife 306 is communicatively coupled to the control unit 302 to receive a termination signal to cut or remove the sensing area 206 of the dipstick 202.

Referring to FIG. 4, a top view of the device 102 shown in Fig. 1, in accordance with aspects of the present specification is depicted. The top surface of the housing 112 has a slot 402 that guides the dipstick 202 to pass over the housing 112. When the user is detected, the sensing area 206 of the dipstick rolls out of the cassette 116 and enters the first end 404 of the slot 402. Further, the dipstick 202 is rolled until the complete strip of sensing area 206 is positioned on the slot 402 to receive the urine flow from the user. The top surface 308 also has shakers 312 placed at regular intervals on the slot 402 to position and/or hold the dipstick 202 on the top surface 308 of the housing 112. Also, the shakers 312 aid in removing excess urine deposited on the dipstick 202. Further, the sensor unit 304 is positioned at the second end 406 opposite to the first end 404 to readout the sensing area 206 of the dipstick 202. More specifically, after the urine flow is terminated, the dipstick 202 is slowly rolled so that the sensing area 304 on the slot is moved out through the second end 406 of the slot 402. While the sensing area 206 is exiting the slot 402, the sensor unit 304 reads the values of the sensing area 206 of the dipstick 202. In one embodiment, each sensing surface of the sensing area 206 may be paused for a predetermined time at the sensor unit 304 so that the sensor unit 304 can read or measure the values. After the readout of all the sensing surfaces, the strip having the sensing area 206 is completely moved out of the slot 402 at the second end 406. Thereafter, the retractable knife 306 cuts the strip of sensing area 206 from the dipstick 202. The removed portion of the sensing area 206 may be collected in the toilet bowl 108. As the strip is biodegradable, it can wash away with the flushing water.

Moreover, while the sensing area 206 is exiting the slot 402, the non-sensing area 208 that is next to the sensing area 206 rolls out of the cassette 116 and it is positioned on the slot 402 until the next usage or urine flow is detected. This in-turn allows the further sensing area that is followed by the non-sensing area 208 to remain dry inside the cassette 116.

The major advantage of this invention is that the dipstick is automatically read out every time a user uses the toilet. As such, the user would be able to perform urinalysis by simply hanging the device 102 inside the toilet bowl and urinate over the device 102. The data will be automatically read out to prevent user dependance and sent to a phone or other digital device to visualize their health status. Also, the dipstick can stay in the toilet for a longer time and measure every time the subject uses the toilet. No need to hold the dipstick anymore whilst user urinates or pee over it. Also, there is no need to manually compare the color of the dipstick to the reference colors on the bottle for readout. Thus, urinalysis is performed in an easy, user independent and automated way.

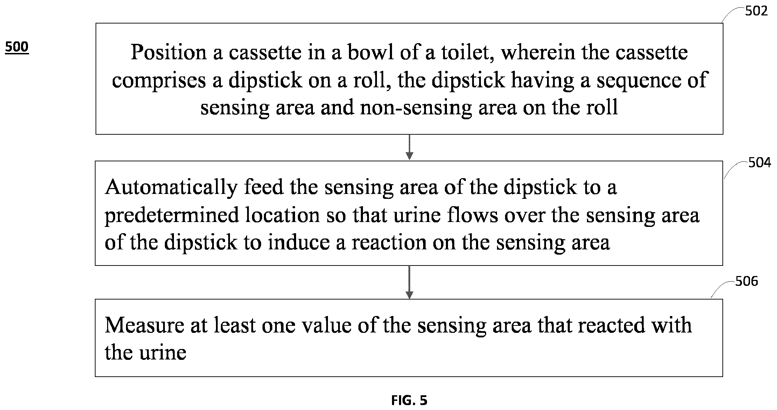

Finally, Fig. 5 shows a flow chart 500 of an embodiment of the inventive method for using the device 102 for urinalysis according to the first aspect of the invention as explained above. It may be noted that the method is described with reference to the components shown in FIGs. 1-4. In step 502, a cassette 116 is positioned in a bowl 108 of a toilet 104. The cassette 116 includes a dipstick 202 on a roll 204. Also, the dipstick 202 has a sequence of sensing area 206 and non-sensing area 208 on the roll 204 as depicted in FIG. 2. Further, at step 504, the sensing area of the dipstick is automatically feed to a predetermined location so that urine flows over the sensing area of the dipstick 202 to induce a reaction on the sensing area 206. Based on the content of the urine, one or more values of the sensing surfaces 210 of the sensing area 206 is changed. These values of the sensing area are measured by the sensor unit 304 of the device 102 as depicted in step 506. Further, the measured values of the sensing area are transmitted to the external device 106.

On the other hand, the external device 106 receives at least one measured value of the sensing area 206 that is in contact with the urine. Further, the external device 106 determines one or more parameters associated with the subject based on the received at least one measured value of the sensing area 206. As depicted in FIG. 1, the data transceiver 122 is communicatively coupled to the device 102 in the toilet to receive the measured one or more values of the sensing area 206. Further, these measured values are transmitted to the processor 124. Thereafter, the processor 124 compares the measured values with a set of reference values stored in the data storage unit 126 to determine one or more parameters of the subject. Based on the variation or deviation in the values of these parameters, one or more markers indicating diseases of the subject is detected. In addition, these markers and related disease information can be communicated to the subject.

While various embodiments of the present invention have been described above, it should be understood that they have been presented by way of example only, and not limitation. Numerous changes to the disclosed embodiments can be made in accordance with the disclosure herein without departing from the spirit or scope of the invention. Thus, the breadth and scope of the present invention should not be limited by any of the above-described embodiments. Rather, the scope of the invention should be defined in accordance with the following claims and their equivalents.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. A device (102) for urinalysis in a bowl of a toilet (104), the device comprising:
a cassette (116) comprising a dipstick (202) on a roll (204), the dipstick (202) having a sequence of sensing area (206) and non-sensing area (208) on the roll;
a control unit (302) operatively coupled to the cassette (116), and configured to automatically feed the sensing area (206) of the dipstick (202) to a predetermined location so that urine flows over the sensing area (206) of the dipstick (202) to induce a reaction on the sensing area (206); and
a sensor unit (304) configured to measure at least one value of the sensing area (206) that reacted with the urine and transmit the measured at least one value to an external device (106).

2. The device (102) according to claim 1, further comprising a holder (110) configured to detachably couple the device (102) to the bowl (108) of the toilet (104), the holder (110) being adjustable to position the cassette (116) to a desired location inside the bowl (108).

3. The device (102) according to claim 1 or 2, wherein the sensor unit (304) comprises at least a color-sensor configured to measure a color value of one or more sensing surfaces (210) on the sensing area (206) of the dipstick (202), wherein the measured color value is included in the at least one value of the sensing area (206) transmitted to the external device (106).

4. The device (102) according to any of the preceding claims, further comprising a housing (112) enclosing the cassette (116), the control unit (302), and the sensor unit (304); and wherein the housing (112) comprises a closing valve (310) configured to allow the dipstick (202) to roll out of the cassette (116) to the predetermined location and prevent urine or water to flow into the housing (112).

5. The device (102) according to any of the preceding claims, further comprising at least one shaker (312) configured to remove excess urine on the sensing area (206) of the dipstick (202) at the predetermined location.

6. The device (102) according to any of preceding claims, further comprising a retractable knife (306) configured to cut off the sensing area (206) of the dipstick (202) after the at least one value of the sensing area (206) is measured by the sensor unit (304).

7. The device (102) according to claim 6, wherein the control unit (302) is operatively coupled to an auto-detect sensor (120), and configured to receive a detection signal from the auto-detect sensor (120), wherein the detector signal indicates the presence of the subject on the toilet (104), wherein the control unit (302) feeds the sensing area (206) of the dipstick (202) to the predetermined location if the detection signal is received, and wherein the control unit (302) comprises an actuator configured to rotate the roll (204) so that the sensing area (206) of the dipstick (202) is fed to the predetermined location.

8. The device (102) according to claim 7, wherein the control unit (302) is operatively coupled to the sensor unit (304), and configured to receive a sensed signal from the sensor unit (304), wherein the sensed signal indicates completion of the measurement of the at least one value of the sensing area (206);
wherein the control unit (302), in response to the received sensed signal, feeds the non-sensing area (208) of the dipstick (202) to the predetermined location and the sensing area (206) of the dipstick towards the retractable knife (306); and
wherein the control unit (302) sends a termination signal to the retractable knife (306) to cut off the sensing area (206) of the dipstick (202) when the sensing area (206) is moved towards the retractable knife (306).

9. The device (102) according to claim 8, wherein the control unit (302) feeds further sensing area of the dipstick (202) to the predetermined location when another detector signal indicating the presence of the subject on the toilet (104) is received from the auto-detector (120), wherein the further sensing area is next to the non-sensing area (208) of the dipstick (202).

10. The device (102) according to any of claims 1 to 7, wherein the control unit (302) detects urine flow from the subject into the bowl of the toilet (104), and wherein the control unit (302) feeds the sensing area (206) of the dipstick to the predetermined location if the urine flow is detected.

11. The device (102) according to claim 5, wherein the sensing area (206) of the dipstick (202) is rolled out of the cassette (116) towards a top surface of the housing (112) that is exposed to the urine flow of the subject, wherein the at least one shaker (312) is positioned on the top surface of the housing (112) to hold and shake the sensing area of the dipstick (202) at the predetermined location.

12. The device (120) according to any of the preceding claims, wherein the sensing area (206) of the dipstick (202) comprises a plurality of sensing surfaces (210), each sensing surface having a color value different from the other sensing surface, wherein each sensing surface changes the color value based on the reaction of the urine with the sensing area (206).

13. A system (100) comprising:
a device (102) according to any of claims 1 to 12; and
an external device (106) communicatively coupled to the device (102), the external device (106) comprising:
a data transceiver (122) configured to receive the at least one value of the sensing area (206) from the sensor unit (304);
a processor (124) coupled to the data transceiver (122), and configured to compare the at least one value of the sensing area (206) with a predetermined dipstick values to determine one or more parameters associated with the subject; and
a display unit (128) configured to display the determined one or more parameters associated with the subject.

14. A method for urinalysis in a bowl of a toilet (104) using a device (102) according to any of the claims 1 to 12, the method comprising the steps of:
positioning a cassette (116) in a bowl of a toilet (104), wherein the cassette (116) comprises a dipstick (202) on a roll (204), the dipstick (202) having a sequence of sensing area (206) and non-sensing area (208) on the roll;
automatically feeding the sensing area (206) of the dipstick (202) to a predetermined location so that urine flows over the sensing area (206) of the dipstick (202) to induce a reaction on the sensing area (206); and
measuring at least one value of the sensing area (206) that reacted with the urine.

15. The method according to claim 14 further comprising:
receiving at least one measured value of the sensing area (206) that is in contact with the urine; and
determining one or more parameters associated with the subject based on the received at least one measured value of the sensing area (206).
